# EUROPEAN PATENT APPLICATION

(11) **EP 4 631 546 A1**
(43) Date of publication of application: **15.10.2025**
(21) Application number: 23901017.6
(22) Date of filing: 01.12.2023
(51) Int. Cl.: A61M 3/02, A61M 39/24

(54) **ENEMA DEVICE AND METHOD OF OPERATING SAME**

(30) Priority: 05.12.2022 KR 20220168149
(71) Applicant: C&Q Medical Co., Ltd., Goyang-si, Gyeonggi-do 10264 (KR)
(72) Inventor: LEE, Ji Min, Seoul 05229 (KR)
(74) Representative: Viering, Jentschura & Partner mbB Patent- und Rechtsanwälte
(86) International application number: PCT/KR2023/019648
(87) International publication number: WO 2024/122998

(57) **Abstract**

Provided are an enema device capable of processing the injection of an enema medicine and the extraction of excrement simultaneously while preventing the excrement from leaking to the outside, and a method of operating the enema device. The enema device includes i) an inner insertion tube including a support rod which extends in one direction to be inserted into an anus and has a hollow interior, and ii) an outer insertion tube which is attachable to or detachable from the inner insertion tube, and is adapted to extend in the one direction to allow the support rod to be inserted thereinto and surround the support rod. The outer insertion tube includes i) a cylindrical tube having a strength lower than the strength of the support rod, and ii) a check valve positioned inside the outer insertion tube.

## Description

### [Technical Field]

The present invention relates to an enema device and a method of operating the same. More specifically, the present invention relates to an enema device capable of processing the injection of an enema medicine and the extraction of excrement simultaneously while preventing the excrement from leaking to the outside, and a method of operating the enema device.

### [Background Art]

An enema refers to a procedure in which an enema liquid is injected into the colon to expel the intestinal contents for the purpose of relieving constipation or removing excrement during surgical procedures or delivery. In general, an enema is performed in cases where severe constipation causes abdominal pain, or in preparation for colon-related procedures such as the diagnosis or surgery of colonic diseases, or colonoscopy for patients with hepatic encephalopathy.

However, in critically ill patients such as those with hepatic encephalopathy, self-administration of an enema is difficult, and thus the procedure must inevitably be performed by caregivers or nurses. The enema procedure involves the insertion of an enema medicine and the subsequent extraction of excrement, each requiring a separate procedure, which has been inconvenient. In addition, due to the weight of critically ill patients, it was difficult to position them properly for enema administration. Furthermore, during the enema procedure, there was a problem of excrement leaking outside the patient's body.

The technology that forms the background of the present invention is disclosed in International Laid-open Patent No. WO2010/122537 and Korean Laid-open Patent No. 2016-0117168.

### [Detailed Description of Invention]

### [Technical Problem]

The present invention provides an enema device capable of processing the injection of an enema medicine and the extraction of excrement simultaneously while preventing the excrement from leaking to the outside. In addition, the present invention provides a method for operating the enema device.

### [Technical Solution]

An enema device according to one embodiment of the present invention includes i) an inner insertion tube including a support rod which extends in one direction to be inserted into an anus and has a hollow interior, and ii) an outer insertion tube which is attachable to or detachable from the inner insertion tube, and is adapted to extend in the one direction to allow the support rod to be inserted thereinto and surround the support rod. The outer insertion tube includes i) a cylindrical tube having a strength lower than the strength of the support rod, and ii) a check valve positioned inside the outer insertion tube.

The cylindrical tube may be made of silicone, and the support rod may be made of polypropylene. The check valve may include a plurality of check valve pieces which are mutually adjacent to each other, and the plurality of check valve pieces may form a cross-shaped opening/closing groove. At least one of the plurality of check valve pieces may include i) a first inclined surface, and ii) a second inclined surface connected to the first inclined surface and formed symmetrically with the first inclined surface. One end of the first inclined surface and one end of the second inclined surface may be each formed in a linear shape to form the opening/closing groove, and the other end of the first inclined surface and the other end of the second inclined surface may be each formed in a curved shape. The first inclined surface and the second inclined surface may form a concave pocket space toward the cylindrical tube.

The outer insertion tube may further include: i) a balloon adapted to inflate while surrounding a mid-side portion of the cylindrical tube and adapted to block the anus; ii) a plate-shaped member connected to one end of the cylindrical tube and having a diameter larger than a diameter of the cylindrical tube; iii) a check valve casing positioned opposite to the cylindrical tube with the plate-shaped member interposed therebetween and having a diameter larger than the diameter of the cylindrical tube and smaller than the diameter of the plate-shaped member; iv) a guide ring including a guide side portion attached to a lower portion of the check valve casing to form a first cavity whose diameter gradually decreases toward the check valve; and v) a fastening member covering the check valve casing and the guide ring, having a protrusion formed on a side surface thereof, and having a through-hole formed at a center thereof.

The check valve may include i) a hollow check valve support plate attached to the lower portion of the check valve casing; ii) a side surface connected to the check valve support plate and forming a second cavity whose diameter gradually decreases toward the cylindrical tube; and iii) a plurality of check valve pieces, which are mutually adjacent to each other, formed on the side, and. The plurality of check valve pieces may form a cross-shaped opening/closing groove.

The inner insertion tube may further include i) a mounting plate to which the support rod is connected and on which the support rod is mounted; and ii) a side portion that is spaced apart from the support rod and surrounds a circumference of the mounting plate. The side surface portion may include i) a first guide hole that is formed open in the one direction; and ii) a second guide hole that is connected to the first guide hole and formed in a direction orthogonal to the first guide hole. A convex portion that protrudes toward the second guide hole in the one direction and is adapted to secure the protrusion may be positioned in the second guide hole. An inner diameter of the side portion may be larger than an outer surface diameter of the fastening member. The inner insertion tube may further include a plurality of gripping columns protruding outward from the side portion and spaced apart from each other at equal intervals, and the first guide hole and the second guide hole may be positioned between the plurality of gripping columns.

The enema device according to one embodiment of the present invention may further include a discharge tube that is attachable to and detachable from the outer insertion tube and includes a flexible discharge portion that is elongated in the one direction and communicates with the cylindrical tube. The discharge tube may further include i) another mounting plate identical to the mounting plate; ii) another side portion identical to the side portion; and iii) a fastening cover coupled to the other side of the flexible discharge portion and having screw threads formed on an inner surface thereof. The other side portion may include i) another first guide hole identical to the first guide hole; and ii) another second guide hole connected to the other first guide hole and identical to the second guide hole. Another convex portion identical to the convex portion and adapted to secure the protrusion may be positioned in the other second guide hole.

The enema device according to one embodiment of the present invention may further include a storage tank detachably coupled to the discharge tube. The reservoir may include: i) an injection port formed on an inner side of the fastening cover and formed with other screw threads adapted to be screw-coupled to the fastening cover by the screw threads; ii) a cap detachably attached to the injection port and adapted to open and close the injection port; and iii) a vinyl bag connected to the injection port. A through-hole in contact with an outer surface of the check valve casing may be formed in the plate-shaped member. The enema device according to one embodiment of the present invention may further include: i) an air injection tube inserted through the through-hole and having an air injection port coupled to one end thereof; and ii) a needle communicating with the other end of the air injection tube and inserted between the balloon and the cylindrical tube. A diameter of the air injection tube may be larger than a diameter of the needle.

The enema device according to one embodiment of the present invention includes a tube that is adapted to communicate with the anus and extends in one direction and has a hollow interior. The enema device further includes a check valve positioned inside the tube. The check valve includes a plurality of check valve pieces which are mutually adjacent to each other, and the plurality of check valve pieces forms a cross-shaped opening/closing groove. At least one of the plurality of check valve pieces includes i) a first inclined surface; and ii) a second inclined surface connected to the first inclined surface and formed symmetrically with the first inclined surface. One end of the first inclined surface and one end of the second inclined surface are each formed in a linear shape to form the opening/closing groove, and the other end of the first inclined surface and the other end of the second inclined surface are each formed in a curved shape. The first inclined surface and the second inclined surface form a concave pocket space toward the anus.

The enema device according to one embodiment of the present invention includes a tube that is adapted to communicate with the anus and extends in one direction and has a hollow interior. The enema device further includes a check valve positioned inside the tube. The check valve includes a plurality of check valve pieces which are mutually adjacent to each other, and the plurality of check valve pieces forms a cross-shaped opening/closing groove. At least one of the plurality of check valve pieces includes i) a first inclined surface, and ii) a second inclined surface connected to the first inclined surface and formed symmetrically with the first inclined surface. One end of the first inclined surface and one end of the second inclined surface are each formed in a linear shape to form the opening/closing groove, and the other end of the first inclined surface and the other end of the second inclined surface are each formed in a curved shape. The first inclined surface and the second inclined surface form a concave pocket space toward the anus.

A method of operating the enema device according to one embodiment of the present invention may include i) inserting the inner insertion tube into the outer insertion tube, and rotating the side portion to sequentially guide the protrusion into the first guide hole and the second guide hole, and securing the protrusion with the convex portion; ii) injecting air into the balloon to inflate the balloon; and iii) removing the inner insertion tube from the outer insertion tube.

The method of operating the enema device according to one embodiment of the present invention may further include i) injecting a syringe through the check valve to inject an enema liquid, and then removing the syringe; and ii) coupling the flexible discharge portion with the cylindrical tube. The discharge tube may further include i) another mounting plate identical to the mounting plate; ii) another side portion identical to the side portion; and iii) a fastening cover coupled to the other side of the flexible discharge portion and having screw threads formed on an inner surface thereof. The another side portion may include i) another first guide hole identical to the first guide hole; and ii) another second guide hole connected to the another first guide hole and identical to the second guide hole. Another convex portion identical to the convex portion may be positioned in the another second guide hole. The coupling of the flexible discharge portion with the cylindrical tube may involve rotating the another side portion to sequentially guide the protrusion into the another first guide hole and the another second guide hole, and securing the protrusion with the other convex portion. The enema device according to one embodiment of the present invention may further include a storage member detachably coupled to the discharge tube. The storage member may include: i) an injection port formed on an inner side of the fastening cover and formed with other screw threads adapted to be screw-coupled to the fastening cover by the screw threads; ii) a cap adapted to be detachably attached to the injection port and adapted to open and close the injection port; and iii) a vinyl bag connected to the injection port.

The method of operating the enema device according to one embodiment of the present invention may further include: i) separating the cap from the injection port; and ii) rotating the injection port and coupling the injection port to an inner side of the fastening cover.

### [Advantageous Effects]

During enema procedures for patients, excrement can be prevented from leaking while allowing for a simple and convenient administration of the enema. Accordingly, the workload of caregivers or nurses in hospitals or nursing facilities can be reduced.

### [Description of Drawings]

FIG. 1 is a schematic exploded perspective view of an enema device according to one embodiment of the present invention.
FIG. 2 is a schematic exploded perspective view of an outer insertion tube included in the enema device in FIG. 1.
FIG. 3 is a schematic perspective view of a check valve in FIG. 2.
FIG. 4 is a schematic drawing of a discharge tube and a storage tank that can be included in the enema device in FIG. 1.
FIG. 5 is a schematic flowchart of a method of operating the enema device in FIG. 1.
FIG. 6 to FIG. 12 are schematic drawings of the process of operating the enema device of the operations in FIG. 5.

### [Modes of the Invention]

Hereinafter, embodiments of the present disclosure will be described in detail with reference to the accompanying drawings so that those skilled in the art to which the present disclosure pertains may readily carry out the invention. However, the present disclosure may be embodied in many different forms and should not be construed as limited to the embodiments described herein. Parts irrelevant to the description are omitted in order to clearly describe the present disclosure in the drawings, and like reference numerals are used to refer to like elements throughout the specification.

In the specification, when a component is said to "include" a certain element, unless explicitly stated otherwise, it does not exclude other elements but may further include additional elements. Devices forming a network may be implemented in hardware, software, or a combination of hardware and software.

As used herein, the term "adapted" shall be interpreted to include both a state in which a component is in proper use and a state before it is in proper use. Moreover, unless explicitly stated otherwise, expressions in the singular form may be interpreted as plural forms as well.

In the present specification, terms including ordinal numbers such as "first," "second," and the like may be used to describe various elements, but the elements should not be limited by such terms. These terms are used only to distinguish one element from another. For example, without departing from the scope of the present disclosure, a first component may be referred to as a second component, and similarly, a second component may be referred to as a first component. In the flowcharts described with reference to the drawings in this specification, the order of operations may be changed, multiple operations may be combined, certain operations may be divided, or specific operations may be omitted.

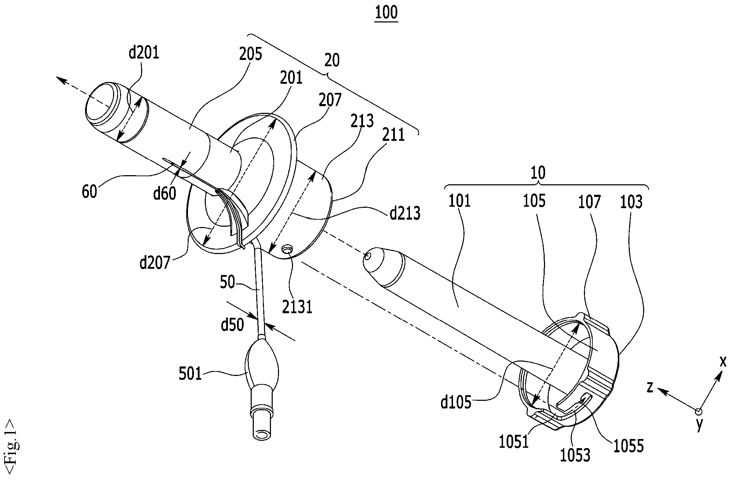

FIG. 1 schematically illustrates an exploded perspective view of an enema device 100 according to one embodiment of the present invention. The structure of the enema device 100 in FIG. 1 is merely for illustrating the present invention, and the present invention is not limited thereto. Therefore, the structure of the enema device 100 in FIG. 1 may be modified differently.

As illustrated in FIG. 1, the enema device 100 includes an inner insertion tube 10 and an outer insertion tube 20. Both the inner insertion tube 10 and the outer insertion tube 20 are formed in a tube form. The inner insertion tube 10 includes a support rod 101. The support rod 101 extends in one direction, i.e., in the z-axis direction, to be inserted into the anus. The support rod 101 may have a hollow interior.

The outer insertion tube 20 is detachably attached to the exterior of the inner insertion tube 10. The outer insertion tube 20 extends in the z-axis direction. As a result, the support rod 101 is inserted along a dotted line into the interior of the outer insertion tube 20, so that the outer insertion tube 20 can surround the support rod 101. The outer insertion tube 20 includes a cylindrical tube 201. The cylindrical tube 201 has a lower strength than the support rod 101. For example, the cylindrical tube 201 may be made of silicone, and the support rod 101 may be made of polypropylene. The outer insertion tube 20 is inserted into the anus for enema administration. The outer insertion tube 20 comes into contact with the anus. Since the cylindrical tube 201 is made of soft silicone, the patient does not experience pain when the outer insertion tube 20 is inserted into the anus. In contrast, if the outer insertion tube 20 were made of a material having a certain strength, the patient could experience pain due to the strength when the outer insertion tube 20 was inserted into the anus. Therefore, the cylindrical tube 201 is made of silicone.

However, since the cylindrical tube 201 is made of a material having relatively low strength, the cylindrical tube 201 may bend easily when inserted into the anus, making it difficult for the outer insertion tube 20 to enter. Therefore, the strength required for the anal insertion of the cylindrical tube 201 can be reinforced by using the support rod 101 inserted into the cylindrical tube 201. That is, the support rod 101 is made of a material having a higher strength than the cylindrical tube 201. As a result, when the outer insertion tube 20 is inserted into the anus for enema administration, the cylindrical tube 201 reinforces the strength of the outer insertion tube 20 and supports the outer insertion tube 20 so that it does not bend, thereby allowing the outer insertion tube 20 to be properly inserted into the anus.

As illustrated in FIG. 1, the inner insertion tube 10 further includes a mounting plate 103, a side portion 105, and gripping columns 107. The support rod 101 is connected to and mounted on the mounting plate 103. Therefore, the support rod 101 can be stably supported by the mounting plate 103. The mounting plate 103 has a circular shape and is positioned on the xy plane.

The side portion 105 is spaced apart from the support rod 101 in the xy-plane direction. The side portion 105 is formed in the form of a wall surrounding the circumference of the mounting plate 103. A first guide hole 1051 and a second guide hole 1053 are formed in the side portion 105. The first guide hole 1051 is formed open in the one direction, i.e., in the z-axis direction. The second guide hole 1053 is connected to the first guide hole 1051. The second guide hole 1053 is formed in the direction orthogonal to the first guide hole 1051, that is, along the x-axis.

A protrusion 2131 in FIG. 1 is sequentially moved along the first guide hole 1051 and the second guide hole 1053 in the direction of the arrow indicated by the dashed line and is secured to the end of the second guide hole 1053. That is, the protrusion 2131 can be moved to the end of the second guide hole 1053 by rotating the inner insertion tube 10 inserted into the outer insertion tube 20. A convex portion 1055 protruding in the -z-axis direction is positioned in the second guide hole 1053. Therefore, the protrusion 2131 is inserted into the inner side of the convex portion 1055 and stably secured by the convex portion 1055. As a result, the inner insertion tube 10 can be stably coupled to the inside of the outer insertion tube 20. Therefore, since the support rod 101 stably supports the cylindrical tube 201 made of a soft material, the enema device 100 can be easily inserted into the anus.

The gripping columns 107 protrude outward from the side portion 105 and are spaced apart from each other at equal intervals. Therefore, the user of the enema device 100 can easily couple the inner insertion tube 10 to the outer insertion tube 20 by holding the gripping columns 107 with his/her hand and rotating the side portion 105. Meanwhile, to avoid interference, the first guide hole 1051 and the second guide hole 1053 are positioned between the gripping columns 107.

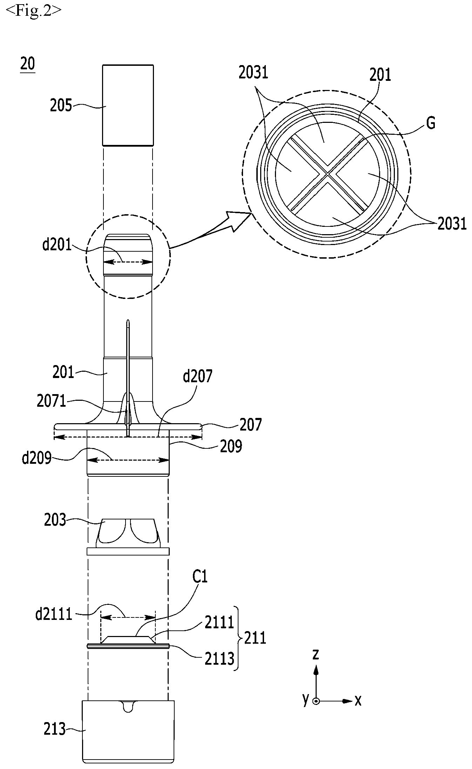

As illustrated in FIG. 1, the outer insertion tube 20 further includes a balloon 205, a plate-shaped member 207, a check valve casing 209 (illustrated in FIG. 2), a guide ring 211, and a fastening member 213. In addition, the outer insertion tube 20 may further include other parts.

The balloon 205 surrounds the mid-side portion of the cylindrical tube 201. The balloon 205 is adapted to be inflated by the injected air to secure the outer insertion tube 20 in the anus. As a result, the outer insertion tube 20 can be stably placed on the anal muscles. For this purpose, the balloon 205 may be formed in a donut shape. A needle 60 is inserted between the balloon 205 and the cylindrical tube 201. The needle 60 communicates with the air injection tube 50.

An air injection port 501 is attached to one end of the air injection tube 50, and the needle 60 communicates with the other end. Although not illustrated in FIG. 1, a syringe is coupled to the air injection port 501, so that air can be injected into the balloon 205 through the air injection tube 50 and the needle 60 using the syringe. The diameter d50 of the air injection tube 50 is larger than the diameter d60 of the needle 60. That is, since a relatively large amount of air is introduced into the air injection tube 50 through the syringe, a volume sufficient to accommodate this is required. Therefore, the diameter d50 of the air injection tube 50 is formed relatively large. Meanwhile, since the needle 60 must be inserted between the balloon 205 and the cylindrical tube 201, its diameter d60 is formed small in consideration of this space.

Meanwhile, the plate-shaped member 207 is connected to one end of the cylindrical tube 201. As the diameter of the cylindrical tube 201 gradually increases, the cylindrical tube 201 is naturally connected to the plate-shaped member 207. The diameter d207 of the plate-shaped member 207 is larger than the diameter d201 of the cylindrical tube 201. Since the diameter d207 of the plate-shaped member 207 is larger than the diameter d201 of the cylindrical tube 201, the plate-shaped member 207 stably supports the cylindrical tube 201.

The fastening member 213 covers the check valve casing 209 (illustrated in FIG. 2) and the guide ring 211 and has a through-hole formed at the center thereof. The protrusion 2131 is formed on the side surface of the fastening member 213. The protrusion 2131 can be inserted into the first guide hole 1051 in the direction of the arrow indicated by the dashed line to stably couple the inner insertion tube 10 and the outer insertion tube 20. To this end, the inner diameter d105 of the side portion 105 is slightly larger than the outer surface diameter d213 of the fastening member 213. For example, the difference between the inner diameter d105 and the outer surface diameter d213 may be greater than 0 and less than 0.2 mm. Since a fine gap is formed in this way, the fastening member 213 can be stably inserted and secured within the side portion 105. Hereinafter, the outer insertion tube 20 will be described in more detail through FIG. 2.

FIG. 2 schematically illustrates the exploded structure of the outer insertion tube 20 included in the enema device 100 in FIG. 1. The enlarged view in FIG. 2 illustrates the front structure of the check valve 203 in the -z-axis direction. The exploded structure of the outer insertion tube 20 in FIG. 2 is merely for illustrating the present invention, and the present invention is not limited thereto. Therefore, the structure of the outer insertion tube 20 in FIG. 2 can be modified differently.

As illustrated in FIG. 2, the outer insertion tube 20 includes a cylindrical tube 201, a check valve 203, a balloon 205, a plate-shaped member 207, a check valve casing 209, a guide ring 211, and a fastening member 213. **In** addition, the outer insertion tube 20 may further include other parts.

The check valve 203 may be made of thin silicone. Therefore, the check valve 203 can be used to block contents inside the body, such as excrement or an enema liquid, from leaking out in the -z-axis direction. The check valve 203 is positioned inside the outer insertion tube 20. More specifically, the check valve 203 is positioned inside the check valve casing 209.

As illustrated in the enlarged view in FIG. 2, the check valve 203 includes check valve pieces 2031 that are adjacent to each other. Since the check valve pieces 2031 are formed separately, they are adjacent to each other to form a cross-shaped opening/closing groove G. However, the cross-shaped opening/closing groove G merely provides a minimum space required for the individual check valve pieces 2031 to move while minimizing mutual contact. Therefore, the cross-shaped opening/closing groove G is too narrow for a fluid with a certain degree of viscosity to flow through. Therefore, the check valve 203 can prevent the contents inside the body or an enema liquid from leaking out in the -z-axis direction. While operating in this manner, since the check valve pieces 2031 are formed separately, the enema liquid can be injected into the body by passing the syringe through the check valve 203.

Furthermore, the check valve 203 has a concave shape in the -z-axis direction, that is, the individual check valve pieces 2031 form pockets. Therefore, the check valve 203 has a shape that blocks the flow of fluid flowing in the -z-axis direction, so that it can block the fluid from passing through.

A through-hole 2071 is formed in the plate-shaped member 207. The through-hole 2071 is formed in contact with the outer surface of the check valve casing 209. That is, since the through-hole 2071 is not easily exposed to the outside and is positioned in contact with the outer surface of the check valve casing 209, the air injection tube 50 (illustrated in FIG. 1) is protected from external impact, thereby ensuring durability.

As illustrated in FIG. 2, the check valve casing 209 is positioned opposite the cylindrical tube 201 with the plate-shaped member 207 interposed therebetween. The check valve casing 209 accommodates the check valve 203. The diameter d209 of the check valve casing 209 is larger than the diameter d201 of the cylindrical tube 201 and smaller than the diameter d207 of the plate-shaped member 207 (illustrated in FIG. 1). Therefore, the check valve casing 209 is stably accommodated under the plate-shaped member 207.

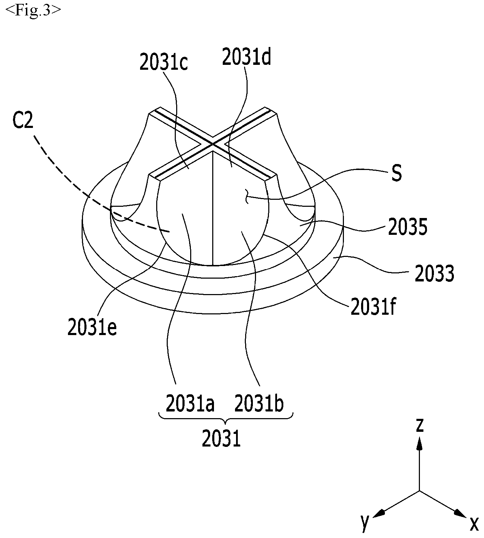

The guide ring 211 is attached to a lower portion of the check valve casing 209. The guide ring 211 includes a guide side portion 2111 and a support plate 2113. The guide side portion 2111 is formed on the support plate 2113. The guide side portion 2111 forms a cavity C1 whose diameter d2111 gradually decreases toward the check valve 203. Hereinafter, the structure of the check valve 203 in FIG. 2 will be described in more detail with reference to FIG. 3.

FIG. 3 schematically illustrates an enlarged view of the check valve 203 in FIG. 2. The structure of the check valve 203 in FIG. 3 is merely for illustrating the present invention, and the present invention is not limited thereto. Accordingly, the structure of the check valve 203 in FIG. 3 may be modified differently. The check valve 203 in FIG. 3 is illustrated as being positioned within the outer insertion tube 20 in FIG. 1 but may be installed within a tube (not illustrated) that is included in the enema device, extends in the one direction, and has a hollow interior. The tube may communicate with the anus.

As illustrated in FIG. 3, each of the check valve pieces 2031 includes a first inclined surface 2031a and a second inclined surface 2031b. The first inclined surface 2031a and the second inclined surface 2031b are formed symmetrically with each other and are open in the +z-axis direction. Therefore, the first inclined surface 2031a and the second inclined surface 2031b have a shape that blocks the flow of fluid that can flow backward in the -z-axis direction.

One end 2031c of the first inclined surface 2031a and one end 2031d of the second inclined surface 2031b are each formed in a linear shape. Therefore, one set of ends 2031c and 2031d of the check valve pieces 2031 meet each other to form a cross-shaped opening/closing groove G. Meanwhile, the other end 2031e of the first inclined surface 2031a and the other end 2031f of the second inclined surface 2031b are each formed in a curved shape. The other set of ends 2031e and 2031f correspond to the circumference inside the cylindrical tube 201. That is, the other set of ends 2031e and 2031f are secured facing the inner wall of the cylindrical tube 201. As a result, the first inclined surface 2031a and the second inclined surface 2031b form a pocket space S that is concave toward the cylindrical tube 201 together with the circumference.

That is, as illustrated in FIG. 3, the pocket space S is formed in the +z-axis direction, i.e., toward the cylindrical tube 201 (illustrated in FIG. 2). The pocket space S functions as a space that can contain an enema liquid or contents inside the body that leak from the anus in case of emergency. As a result, the leakage of these fluids can be prevented by using the check valve 203. The structure of the check valve 203 will now be described in more detail.

The check valve 203 further includes a check valve support plate 2033 and a side surface 2035. The check valve support plate 2033 is formed in a hollow shape. The check valve support plate 2033 is attached to the lower portion of the check valve casing 209 (illustrated in FIG. 2) to form a support structure. The side surface 2035 is connected to the check valve support plate 2033. The diameter of the side surface 2035 gradually decreases in the +x-axis direction, that is, toward the cylindrical tube 201 (illustrated in FIG. 2), forming a cavity C2. The side surface 2035 is formed integrally around the inner circumference of the check valve casing 209. The check valve pieces 2031 are formed on the side surface 2035 and are adjacent to each other. The check valve pieces 2031 may be formed integrally with the side surface 2035. That is, the side surface 2035 may also be made of thin silicone in the same manner as the check valve pieces 2031.

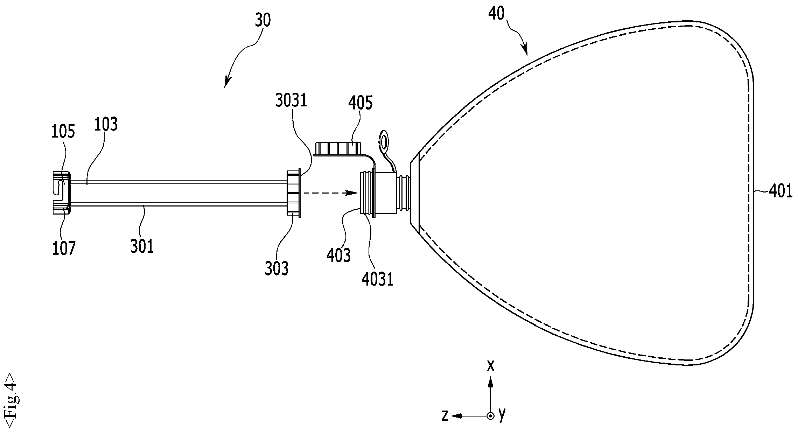

FIG. 4 schematically illustrates the discharge tube 30 and the storage tank 40 included in the enema device 100 in FIG. 1. The discharge tube 30 and the storage tank 40 in FIG. 4 are merely for illustrating the present invention, and the present invention is not limited thereto. Accordingly, the discharge tube 30 and the storage tank 40 may be modified differently. The mounting plate 103, the side portion 105, and the gripping columns 107 included in the discharge tube 30 in FIG. 4 are the same as the mounting plate 103, the side portion 105, and the gripping columns 107 included in the inner insertion tube 10 in FIG. 1. Therefore, the same drawing reference numerals are used, and a detailed description thereof is omitted for convenience. In this way, since the inner insertion tube 10 and the discharge tube 30 use the same parts, not only can the manufacturing cost of the enema device 100 be reduced, but also the inner insertion tube 10 can be detachably attached interchangeably with the outer insertion tube 20 when operating the enema device 100 (illustrated in FIG. 1).

As illustrated in FIG. 4, the discharge tube 30 is elongated in the one direction, that is, in the z-axis direction. The discharge tube 30 includes a flexible discharge portion 301, a fastening cover 303, a mounting plate 103, a side portion 105, and gripping columns 107. In addition, the discharge tube 30 may further include other parts. For example, although not illustrated in FIG. 4, a clip may be coupled to the middle of the flexible discharge portion 301 and the flexible discharge portion 301 may be pressed to restrict the flow of fluid passing through the flexible discharge portion.

The discharge tube 30 is attachable to and detachable from the outer insertion tube 20 (illustrated in FIG. 1) through the side portion 105. That is, by using the side portion 105, the discharge tube can be attached to and detached from the outer insertion tube 20 (illustrated in FIG. 1) in the same way as the inner insertion tube 10 (illustrated in FIG. 1) including the side portion 105. As a result, the flexible discharge portion 301 communicate with the cylindrical tube 201 (illustrated in FIG. 1). Therefore, the contents of the body discharged from the anus through the cylindrical tube 201 (illustrated in FIG. 1), for example, excrement, can be continuously discharged through the flexible discharge portion 301.

A fastening member 213 (illustrated in FIG. 1) is mounted on the mounting plate 103 to stably support the coupling between the outer insertion tube 20 and the discharge tube 30. The mounting plate 103 is coupled to one side of the flexible discharge portion 301, and the fastening cover 303 is coupled to the other side of the flexible discharge portion 301. The side portion 105 is spaced apart from the flexible discharge portion 301. Screw threads 3031 are formed on the inner surface of the fastening cover 303. The discharge tube 30 and the storage tank 40 can be firmly coupled using the screw threads 3031. That is, the storage tank 40 can be attached to and detached from the discharge tube 30 in the direction of the arrow indicated by the dotted line.

As illustrated in FIG. 4, the storage tank 40 includes a vinyl bag 401, an injection port 403, and a cap 405. In addition, the storage tank 40 may further include other parts.

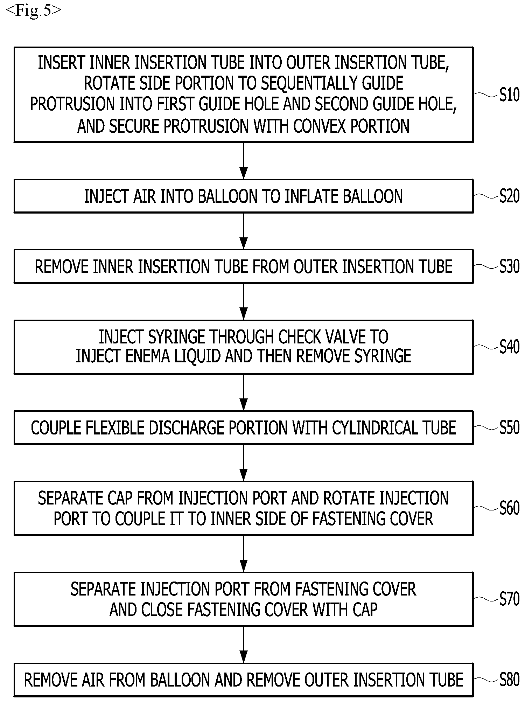

As illustrated in FIG. 4, screw threads 4031 corresponding to the screw threads 3031 formed on the inner side of the fastening cover 303 are formed in the injection port 403. Since the screw threads 3031 and 4031 are formed in male and female configurations and engage with each other, the injection port 403 and the fastening cover 303 are firmly joined together to prevent the leakage of the contents inside the body. The cap 405 can be attached to and detached from the injection port 403. That is, when the vinyl bag 401 coupled to the injection port 403 is not used, the injection port 403 is closed using the cap 405. In addition, when the storage tank 40 is connected to the discharge tube 30 to contain the contents inside the body discharged after the enema administration, the cap 405 is opened and the injection port 403 is fastened to the fastening cover 303. As a result, the contents of the body discharged through the outer insertion tube 20 after the enema administration can be received in the vinyl bag 401 through the discharge tube 30 without leakage. Thereafter, the injection port 403 can be separated from the fastening cover 303 and the cap 405 can be closed, allowing the storage tank to be discarded as is. The method of operating this enema device 100 (illustrated in FIG. 1) will be described in detail below with reference to FIG. 5 to FIG. 12.

FIG. 5 schematically illustrates a flowchart of the method of operating the enema device 100 in FIG. 1. The method of operating the enema device 100 in FIG. 5 is merely for illustrating the present invention, and the present invention is not limited thereto. Therefore, the method of operating the enema device 100 in FIG. 5 may be modified differently.

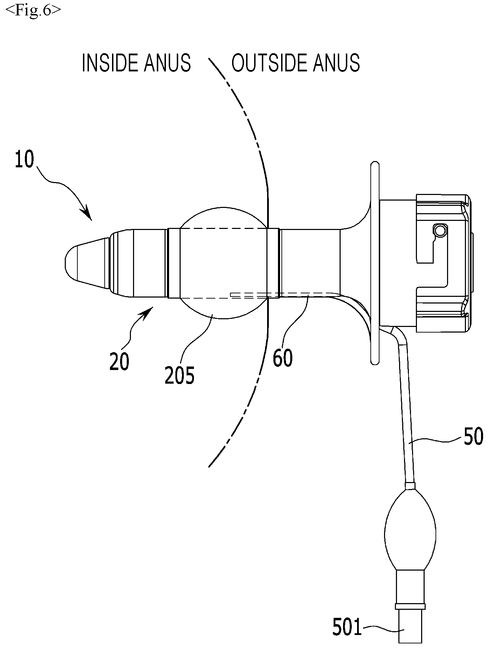

Meanwhile, FIG. 6 to FIG. 12 schematically illustrate the process of operating the enema device at each operation in FIG. 5. Each process of operating the enema device of FIG. 6 to FIG. 12 is merely for illustrating the present invention, and the present invention is not limited thereto. Therefore, each process of operating the enema device can be modified differently. Hereinafter, the method of operating the enema device 100 will be described in detail with reference to FIG. 6 to FIG. 12 corresponding to each operation in FIG. 5.

As illustrated in FIG. 5, the method of operating the enema device 100 includes operation S10 of inserting the inner insertion tube into the outer insertion tube, rotating the side portion to sequentially guide the protrusion into the first guide hole and the second guide hole, and securing the protrusion with the convex portion, operation S20 of injecting air into the balloon to inflate the balloon, operation S30 of removing the inner insertion tube from the outer insertion tube, operation S40 of injecting the syringe through the check valve to inject an enema liquid, and then removing the syringe, operation S50 of coupling the flexible discharge portion with the cylindrical tube, operation S60 of separating the cap from the injection port and rotating the injection port to couple the injection port to the inner side of the fastening cover, operation S70 of separating the injection port from the fastening cover and closing the fastening cover with the cap, and operation S80 of removing air from the balloon and removing the outer insertion tube. In addition, the method of operating the enema device may further include other operations.

First, in operation S10 in FIG. 5, the inner insertion tube 10 is inserted into the outer insertion tube 20, and the side portion 105 is rotated so that the protrusion 2131 is sequentially guided into the first guide hole 1051 and the second guide hole 1053 in the direction of the arrow indicated by the dashed line and secured with the convex portion 1055 (illustrated in FIG. 1). As a result, the inner insertion tube 10 and the outer insertion tube 20 are firmly integrally coupled together. Then, the inner insertion tube 10 and the outer insertion tube 20 are inserted into the anus for enema administration. Since the outer insertion tube 20 includes the cylindrical tube 201 made of silicone, the patient does not experience much pain even when the anus comes into contact with the outer insertion tube 20 when the outer insertion tube 20 is inserted into the anus. Meanwhile, since the cylindrical tube 201 is supported by the support rod 101 made of polypropylene included in the inner insertion tube 10, the cylindrical tube 201 made of silicone is not bent or crushed, and therefore can be easily inserted into the anus.

Returning again to FIG. 5, in operation S20, air is injected into the balloon 205 to inflate the balloon 205 (illustrated in FIG. 6). The balloon 205 is positioned just on the inner side of the anus in an uninflated state. A syringe (not illustrated) is connected to the air injection port 501 and a piston (not illustrated) is pressed to inject air into the balloon 205 through the air injection tube 50 and the needle 60, thereby inflating the balloon 205. As a result, the balloon 205 is inflated into a donut shape, and the outer insertion tube 20 is stably placed on the anal muscles.

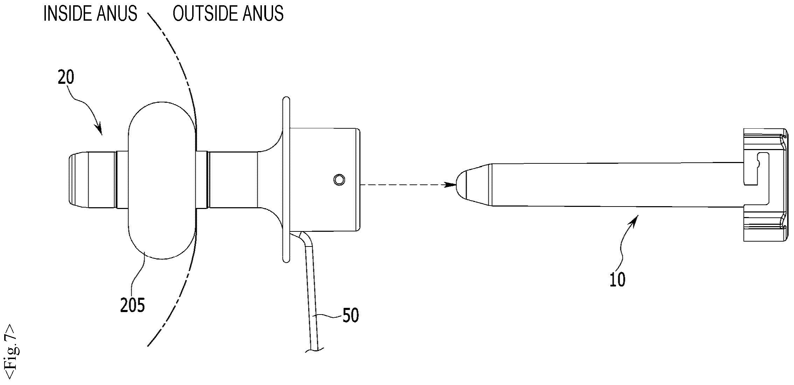

**In** operation S30 in FIG. 5, as indicated by the dotted arrow, the inner insertion tube 10 is removed from the outer insertion tube 20 (illustrated in FIG. 7). That is, the inner insertion tube 10 is removed to secure a space for injecting an enema liquid. The balloon 205 is inflated to stably place the outer insertion tube 20 in the anus.

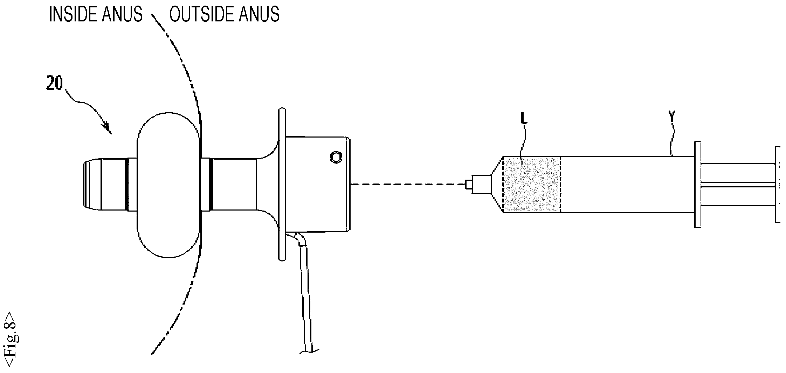

In operation S40 in FIG. 5, the syringe Y is injected through the check valve to inject an enema liquid L into the anus (illustrated in FIG. 8). Then, the syringe Y is removed. The syringe used in operation S20 may be used as the syringe Y. Since the check valve 203 (not illustrated, the same hereinafter) has a convex shape toward the anus, it is easy for the syringe Y to penetrate the check valve 203. In addition, the enema liquid injected into the anus through the outer insertion tube 20 does not leak to the outside due to the check valve 203.

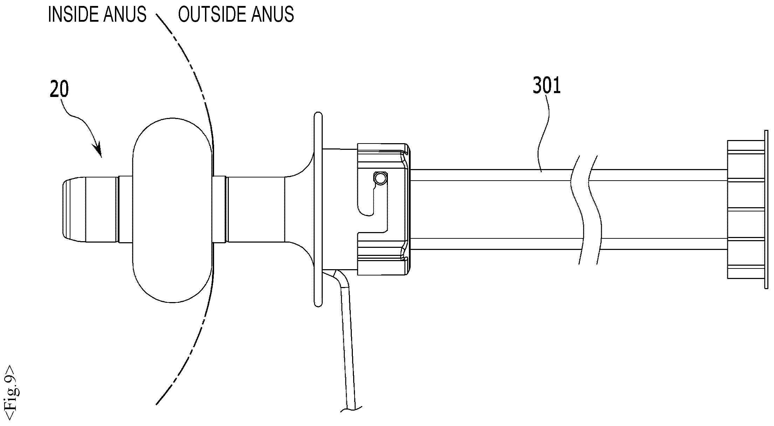

Returning again to FIG. 5, in operation S50, the flexible discharge portion 301 is coupled to the outer insertion tube 20 (illustrated in FIG. 9). Therefore, the bodily contents generated by the enema administration are prepared for discharge through the flexible discharge portion 301. The method of coupling the flexible discharge portion 301 to the outer insertion tube 20 is the same as the coupling method in operation S10.

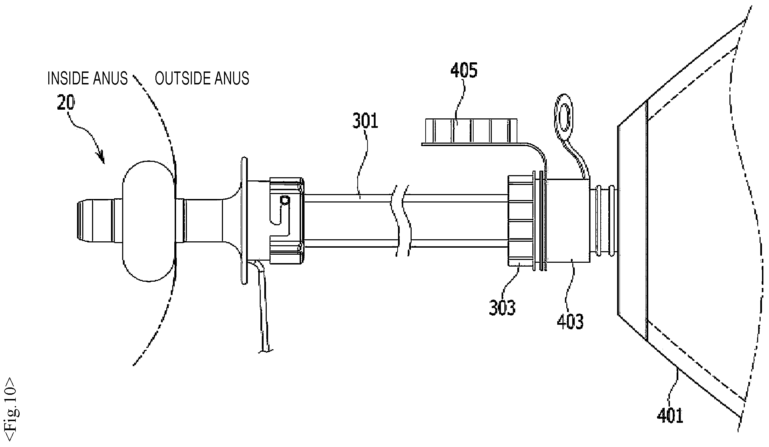

In operation S60 in FIG. 5, the cap 405 is separated from the injection port 403, and the injection port 403 is rotated to couple the injection port 403 to the inner side of the fastening cover 303 (illustrated in FIG. 10). The fastening cover 303 may be rotated and coupled to the injection port 403. As a result, the flexible discharge portion 301 and the injection port 403 are firmly fastened without leakage. Since the flexible discharge portion 301 is in an open state before the injection port 403 is coupled thereto, a clip (not illustrated) may be installed in the middle of the flexible discharge portion 301 to take measures such that the bodily contents discharged through the anus do not leak out by pressing the flexible discharge portion 301. After such measures are taken, the injection port 403 can be coupled to the fastening cover 303. Then, the clip can be removed so that the bodily contents discharged through the flexible discharge portion 301 can be properly contained in the vinyl bag 401.

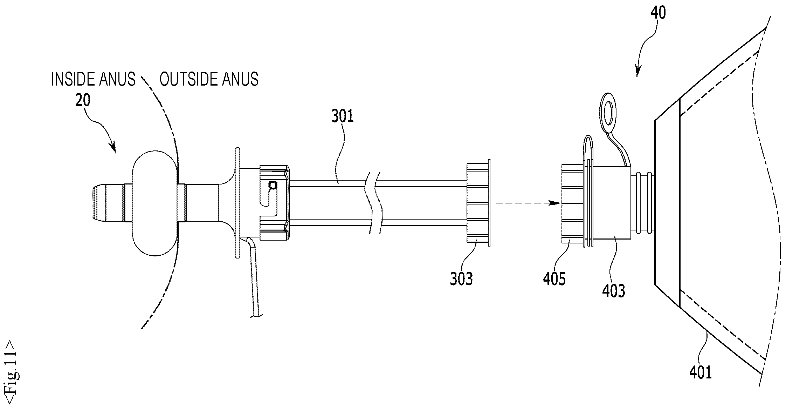

Returning again to FIG. 5, in operation S70, the injection port 403 is separated from the fastening cover 303 and the fastening cover 303 is closed with the cap 405 (indicated by the arrow in FIG. 11). That is, since all the bodily contents discharged by the enema administration are contained in the vinyl bag 401, the cap 405 is closed and the storage tank 40 is discarded.

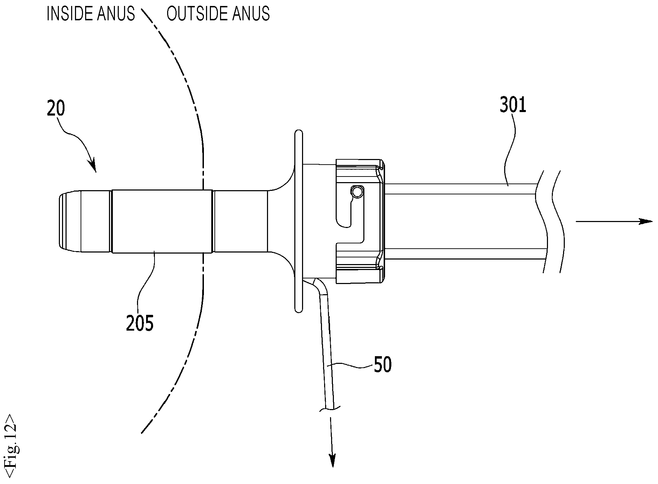

Finally, in operation S80, air is removed from the balloon and the outer insertion tube 20 is removed from the anus (illustrated in FIG. 12). That is, a syringe (not illustrated) is connected to the air injection tube 50 and the air inside the balloon 205 is removed through the air injection tube 50. As a result, the outer insertion tube 20 and the flexible discharge portion 301 connected thereto can be pulled in the direction of the arrow and removed altogether. It is also possible to remove the flexible discharge portion 301 first and then the outer insertion tube 20. However, since the outer insertion tube 20 is made of a soft material and is short in length, it is preferable to pull the flexible discharge portion 301 that is firmly coupled to the outer insertion tube 20 and has an elongated shape so that the outer insertion tube 20 is also withdrawn from the anus together.

The enema can be easily performed through the above-described processes. In addition, since the enema device according to one embodiment of the present invention is manufactured to cover the entire enema process, it is highly convenient to use.

Although embodiments of the present disclosure have been described in detail above, the scope of the present disclosure is not limited thereto, and various modifications and improvements by those skilled in the art using the basic concepts defined in the following claims also fall within the scope of the present disclosure.

## Claims

1. An enema device comprising:
an inner insertion tube including a support rod which extends in one direction to be inserted into an anus and has a hollow interior; and
an outer insertion tube which is attachable to or detachable from the inner insertion tube, and is adapted to extend in the one direction to allow the support rod to be inserted thereinto and surround the support rod,
wherein the outer insertion tube comprises:
a cylindrical tube having a strength lower than the strength of the support rod; and
a check valve positioned inside the outer insertion tube, through which the inner insertion tube passes, and adapted to prevent excrement inside the anus from leaking to the outside.

2. The enema device according to claim 1, wherein the cylindrical tube is made of silicone, and the support rod is made of polypropylene.

3. The enema device according to claim 1, wherein the check valve comprises a plurality of check valve pieces which are mutually adjacent to each other, and the plurality of check valve pieces form a cross-shaped opening/closing groove.

4. The enema device according to claim 3, wherein at least one of the plurality of check valve pieces comprises:
a first inclined surface; and
a second inclined surface connected to the first inclined surface and formed symmetrically with the first inclined surface, and
one end of the first inclined surface and one end of the second inclined surface are each formed in a linear shape to form the opening/closing groove, and the other end of the first inclined surface and the other end of the second inclined surface are each formed in a curved shape.

5. The enema device according to claim 4, wherein the first inclined surface and the second inclined surface form a concave pocket space toward the cylindrical tube.

6. The enema device according to claim 1, wherein the outer insertion tube further comprises:
a balloon adapted to inflate while surrounding a mid-side portion of the cylindrical tube and adapted to block the anus;
a plate-shaped member connected to one end of the cylindrical tube and having a diameter larger than a diameter of the cylindrical tube;
a check valve casing positioned opposite to the cylindrical tube with the plate-shaped member interposed therebetween and having a diameter larger than the diameter of the cylindrical tube and smaller than the diameter of the plate-shaped member;
a guide ring including a guide side portion attached to a lower portion of the check valve casing to form a first cavity whose diameter gradually decreases toward the check valve; and
a fastening member covering the check valve casing and the guide ring, having a protrusion formed on a side surface thereof, and having a through-hole formed at a center thereof,
the check valve further comprises:
a hollow check valve support plate attached to the lower portion of the check valve casing;
a side surface connected to the check valve support plate and forming a second cavity whose diameter gradually decreases toward the cylindrical tube; and
a plurality of check valve pieces, which are mutually adjacent to each other, formed on the side, and
the plurality of check valve pieces form a cross-shaped opening/closing groove.

7. The enema device according to claim 6, wherein the inner insertion tube further comprises:
a mounting plate to which the support rod is connected and on which the support rod is mounted; and
a side portion that is spaced apart from the support rod and surrounds a circumference of the mounting plate,
the side portion includes:
a first guide hole that is formed open in the one direction; and
a second guide hole that is connected to the first guide hole and formed in a direction orthogonal to the first guide hole, and
a convex portion that protrudes toward the second guide hole in the one direction and is adapted to secure the protrusion is positioned in the second guide hole.

8. The enema device according to claim 7, wherein an inner diameter of the side portion is larger than an outer surface diameter of the fastening member,
the inner insertion tube further comprises a plurality of gripping columns protruding outward from the side portion and spaced apart from each other at equal intervals, and
the first guide hole and the second guide hole are positioned between the plurality of gripping columns.

9. The enema device according to claim 7, further comprising a discharge tube that is attachable to and detachable from the outer insertion tube and includes a flexible discharge portion that is elongated in the one direction and communicates with the cylindrical tube,
wherein the discharge tube further comprises:
another mounting plate identical to the mounting plate;
another side portion identical to the side portion; and
a fastening cover coupled to the other side of the flexible discharge portion and having screw threads formed on an inner surface thereof,
the another side portion includes:
another first guide hole identical to the first guide hole; and
another second guide hole connected to the another first guide hole and identical to the second guide hole, and
another convex portion identical to the convex portion and adapted to secure the protrusion is positioned in the another second guide hole.

10. The enema device according to claim 9, further comprising a storage tank detachably coupled to the discharge tube,
wherein the storage tank comprises:
an injection port formed on an inner side of the fastening cover and formed with other screw threads adapted to be screw-coupled to the fastening cover by the screw threads;
a cap detachably attached to the injection port and adapted to open and close the injection port; and
a vinyl bag connected to the injection port.

11. The enema device according to claim 6, wherein a through-hole in contact with an outer surface of the check valve casing is formed in the plate-shaped member,
the enema device further comprises:
an air injection tube inserted through the through-hole and having an air injection port coupled to one end thereof; and
a needle communicating with the other end of the air injection tube and inserted between the balloon and the cylindrical tube, and
wherein a diameter of the air injection tube is larger than a diameter of the needle.

12. The enema device according to claim 1, further comprising a discharge tube that is attachable to and detachable from the outer insertion tube and includes a flexible discharge portion that extends long in the one direction and communicates with the cylindrical tube.

13. A method of operating an enema device that is the enema device according to claim 9, the method comprising:
inserting the inner insertion tube into the outer insertion tube, and rotating the side portion to sequentially guide the protrusion into the first guide hole and the second guide hole, and securing the protrusion with the convex portion;
injecting air into the balloon to inflate the balloon; and
removing the inner insertion tube from the outer insertion tube.

14. The method according to claim 13, further comprising:
injecting a syringe through the check valve to inject an enema liquid, and then removing the syringe; and
coupling the flexible discharge portion with the cylindrical tube.

15. The method according to claim 14, wherein the discharge tube further comprises:
another mounting plate identical to the mounting plate;
another side portion identical to the side portion; and
a fastening cover coupled to the other side of the flexible discharge portion and having screw threads formed on an inner surface thereof,
the another side portion includes:
another first guide hole identical to the first guide hole; and
another second guide hole connected to the another first guide hole and identical to the second guide hole, and
another convex portion identical to the convex portion is positioned in the another second guide hole, and
the coupling of the flexible discharge portion with the cylindrical tube involves rotating the another side portion to sequentially guide the protrusion into the another first guide hole and the another second guide hole, and securing the protrusion with the another convex portion.

16. The method according to claim 15, wherein the enema device further comprises a storage member detachably coupled to the discharge tube,
the storage member comprises:
an injection port formed on an inner side of the fastening cover and formed with other screw threads adapted to be screw-coupled to the fastening cover by the screw threads;
a cap adapted to be detachably attached to the injection port and adapted to open and close the injection port; and
a vinyl bag connected to the injection port, and
the method further comprises:
separating the cap from the injection port; and
rotating the injection port and coupling the injection port to an inner side of the fastening cover.
